# EUROPEAN PATENT SPECIFICATION

(11) **EP 3 946 504 B1**
(45) Date of publication and mention of the grant of the patent: **04.01.2023**
(21) Application number: 20718601.6
(22) Date of filing: 03.04.2020
(51) Int. Cl.: A61M 60/859, A61M 60/861, A61M 60/196

(54) **A VASCULAR COUPLING DEVICE**
GEFÄSSKOPPLUNGSVORRICHTUNG
DISPOSITIF DE RACCORD VASCULAIRE

(30) Priority: 05.04.2019 SE 1950431; 26.08.2019 SE 1950977
(43) Date of publication of application: 09.02.2022
(73) Proprietor: Scandinavian Real Heart AB, 722 13 Västerås (SE)
(72) Inventor: NAJAR, Azad, 723 41 Västerås (SE)
(74) Representative: Valea AB
(86) International application number: PCT/EP2020/059644
(87) International publication number: WO 2020/201545

(56) References cited:
- WO-A1-2016/020219
- US-A1- 2009 204 206

## Description

### TECHNICAL FIELD

The present invention relates to a vascular coupling device for connecting any heart prosthesis such as a total artificial heart (TAH) to the vascular system of a subject in need of a circulatory support system. A method for connecting a TAH to the vascular system of a subject is also disclosed.

### BACKGROUND OF THE INVENTION

The main function of the heart in the human body is to circulate blood through the blood vessels in order to transport oxygen, nutrition, and waste products to and from body cells. Many diseases may affect the heart such as myocardial infarction, hypertension, valve insufficiency and various heart muscle diseases. The end result of such diseases may be heart failure which means that the heart has lost its ability to pump enough blood to the lungs and body tissues.

The symptoms of heart failure are shortness of breath, edema and fatigue. The only treatment option available for a patient suffering from advanced heart failure is heart transplantation. However, due to a lack of sufficient number of donor hearts, the majority of advanced heart failure patients die while waiting for a heart transplant operation.

For this reason many efforts have been made during the last 50 years to develop a mechanical heart which can replace a diseased heart entirely. Until now only a few Total Artificial Hearts (TAH) i.e. mechanical hearts/heart prostheses have been developed which have the capacity to completely replace the diseased heart.

WO2016/020219 discloses a four-chambered TAH which is designed as a human heart. This TAH comprises a first and a second artificial heart pump corresponding to the left and right heart of the natural heart. Each artificial pump comprises an inlet and an outlet channel and a valve cylinder which is divided into two chambers by means of a moving plane comprising a one-way valve which corresponds to the atrioventricular (AV) plane in a natural heart. Pump actuating means are configured to apply a movement to the valve cylinders in an upward and downward direction in response to control signals from a control unit such that when the valve cylinders move in an upward direction inside the blood pump housing device, the valves provided in the valve planes are in an open position allowing a flow of blood through the inlet channel into the artificial atrium, and thereafter into the artificial ventricle. When the valve cylinders move in a downward direction the valves are in the closed position and blood is ejected from the artificial ventricle and exit therefrom through outlet channels.

The TAH is enclosed in a casing which protects the surrounding tissue from moving parts and prevents entry of body fluids into the TAH. When implanted in a subject the diseased natural heart of the subject is removed and thereafter the circulatory system of the subject is connected to the inlets and outlets of the TAH. The coupling device connecting the vascular system with the inlets and outlets of the TAH has to be absolutely leak-proof as well as easy to connect and disconnect from the TAH. Furthermore, the vascular coupling device must have the capacity to elastically accommodate any body movements of the subject without damaging the vascular system of the patient, or disconnecting it from the TAH.

Further relevant prior art is disclosed in document US2009/204206 A1.

### SUMMARY OF THE INVENTION

The above objects may be achieved with a vascular coupling device in accordance with independent claim 1, and a TAH having inlet and outlet channels connected to the vascular coupling device according to independent claim 18. Further embodiments are set out in the dependent claims, the description and in the drawings.

The vascular coupling device as disclosed herein will provide an absolutely leak-proof coupling between the vascular system of the patient and the inlets and outlets of a TAH. The vascular coupling device is easily connected to the circulatory system of the subject, i.e. both the systemic circulation circuit and the pulmonary circulation circuit as well as to the TAH in a leak-proof and flexible manner.

As set out herein, there is provided a vascular coupling device comprising a first and a second coupling element wherein each one of the first and second coupling elements has an external surface facing an external side, a coupling surface facing a coupling side, and a central opening.

The vascular coupling device further comprises a first and a second tubular connecting element, wherein each one of the first and second tubular connecting elements has a first open end and a second open end. The first open ends are provided with flanges. Each one of the first and second tubular connecting elements is arranged in a corresponding central opening of the first and second coupling elements respectively, while the second open ends of the first and second tubular connecting elements protrude through the central openings on the external side of each of the first and second coupling elements.

The first and second coupling elements are removably connected to each other into a locked configuration, or disconnected from each other into an unlocked configuration by means of a first and second locking structure. The first and second locking structures are arranged on a centerline A and opposite to each other and on an outer perimeter of the vascular coupling device.

The vascular device further comprises a fail-safe arrangement comprising first and second cut-in portions arranged on said first coupling element configured to receive first and second projecting elements arranged on said second coupling element, thereby preventing erroneous connection of said first and second coupling elements to each other.

The inner perimeters of the central openings are advantageously provided with grooves arranged facing the coupling sides of the coupling elements, and the flanges of the first open ends of the first and second tubular connecting elements are advantageously provided with upwardly turned lips. Advantageously the flanges with upwardly turned lips rest in the grooves of the central opening to provide a secure connection of the tubular connecting element to the first and second coupling elements.

Each one of the first and second locking structure comprises a female locking part arranged on the first coupling element, a male locking part arranged on the second coupling element and a locking element configured to join the female locking part with the male locking part into a locked configuration wherein the coupling surfaces of the first and second coupling elements are brought into contact such that the first open ends of the first and second tubular connecting elements are brought into a sealing connection.

Each one of the female locking parts comprises a first and a second lug having a gap therebetween, and the first and second lugs are arranged on opposite sides B and C of the centerline A. The first lug arranged on the side B has a first bore and the second lug arranged on the side C has a second bore, wherein the second bore is provided with an inner screw thread.

Each one of the male locking parts is arranged on the centerline A and is configured to be received into the gap between the first and second lugs of the female locking parts.

Each one of the locking elements has a first end provided with an outer screw thread, a second end provided with a cone shaped head, and a mid-section arranged between the first and second ends. The locking elements are configured to be received into the first and second bores of the female locking part.

Both of the locking elements are arranged to be entered into the first bores from a first side B and perpendicular to the centerline A, cross the centerline A and thereafter be received into the second bores provided with screw threads and arranged on a second side C of the centerline A, and opposite to the first side B.

Each one of the male locking parts comprises an eye provided with an open cleft facing the coupling side of the second coupling element.

The mid-sections of the locking elements are configured to be received into the open clefts provided on the eyes of the male locking parts when the male locking parts are arranged in the gaps between the first and second lugs of the female locking parts.

First and second recesses are arranged around inner perimeters of the eyes and on same side of said centerline A of the male locking parts. The first and second recesses are configured to receive the cone-shaped heads of the locking elements.

When the vascular device is in a locked configuration, the outer screw threads of the locking elements are received into and joined with the inner screw threads provided in the second bores of the female locking parts, and the cone shaped heads of the locking elements are received into the recesses provided on the eyes arranged on the male locking parts.

The first and second projecting elements are arranged on an outer perimeter of the second coupling element on the same side of the centerline A.

The first and second cut-in portions are arranged on an outer perimeter and on the same side of the centerline A on the first coupling element.

An encasing sac is attached to the external surfaces of the coupling device and receives and encloses the artificial heart pump to protect the pump from tissue ingrowth.

A first and second receiving means are arranged on the first and second coupling elements respectively, both first and second receiving means being arranged on the same side of the center line A and are adapted to facilitate manipulation of said vascular coupling device during implantation of the vascular coupling device.

The vascular coupling device as described herein is advantageously connected to one or more of the first and second inlet and outlet channels of a total artificial heart.

### BRIEF DESCRIPTION OF THE DRAWINGS

Figures 1A-D disclose the vascular coupling device A) in an exploded configuration, B) in an unlocked configuration, C) in a connected configuration and D) in a connected and locked configuration.
Figures 2A and B disclose the first and second coupling elements as viewed from the coupling sides.
Figures 3A and B are views of the first and second tubular connecting elements.
Figures 4A-C are cross-sectional views of a vascular coupling device in A) in an exploded configuration, B) in an unlocked configuration and C) in a connected configuration.
Figures 5A-d are views of the vascular coupling device when connected to a total artificial heart.
Figures 6A-D are views of A) the locking element, B) the locking element in a vascular coupling device when in an unlocked configuration, C) the locking element in a vascular coupling device when in a connected configuration and D) the locking in a vascular coupling device when in a locked configuration.
Figures 7A and B are views of the vascular coupling device when connected to a total artificial heart.
Figure 8 is a view of an encasing sac enclosing the total artificial heart.
Figure 9 discloses the receiving means adapted for receiving holding means for holding and manipulating the vascular coupling device during the implantation process.
Figures 10A and 10B disclose the vascular coupling device wherein holding means for holding and manipulating the vascular coupling device during the implantation process are attached.

### DETAILED DESCRIPTION

In the following detailed description, reference is made to the accompanying set of drawings that form a part of the description hereof and in which several specific embodiments are shown by way of illustration. It is to be understood that other embodiments are contemplated and may be made without departing from the scope of the present invention as defined by the appended claims.

The following detailed description, therefore, is not to be taken in a limiting sense.

The terms "a", "an", and "the" include plural referents unless the content clearly dictates otherwise. The term "or" is generally employed in its sense including "and/or" unless the content clearly dictates otherwise.

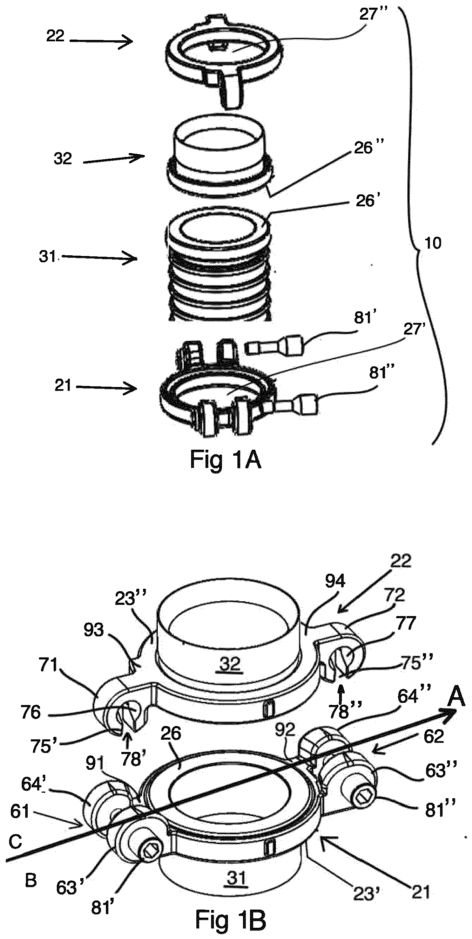

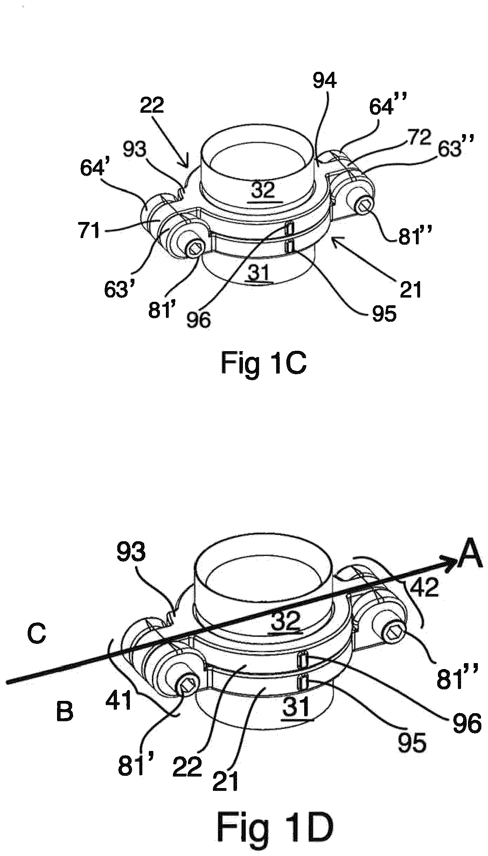

Figures 1-4 disclose a vascular coupling device 10 for connecting an artificial heart pump of a TAH to the vascular system of a subject. The vascular coupling device 10 is easily connected and/or disconnected to the artificial heart pump and forms a leak-proof and safe conduit between the patient's vascular system and the artificial heart pump.

The vascular coupling device 10 comprises a first and a second coupling element 21, 22, a first and second tubular connecting element 31, 32 and a first and second locking structure 41, 42, arranged to reversibly connect or disconnect the first and second coupling elements 21, 22 into a locked or unlocked configuration (see Figs. 1A-D). A centerline A for the vascular coupling device 10 extends through the pair of first and second locking structures 41, 42 which are arranged on the centerline A and opposite to each other on an outer perimeter of said vascular coupling device 10 (see Fig. 1D).

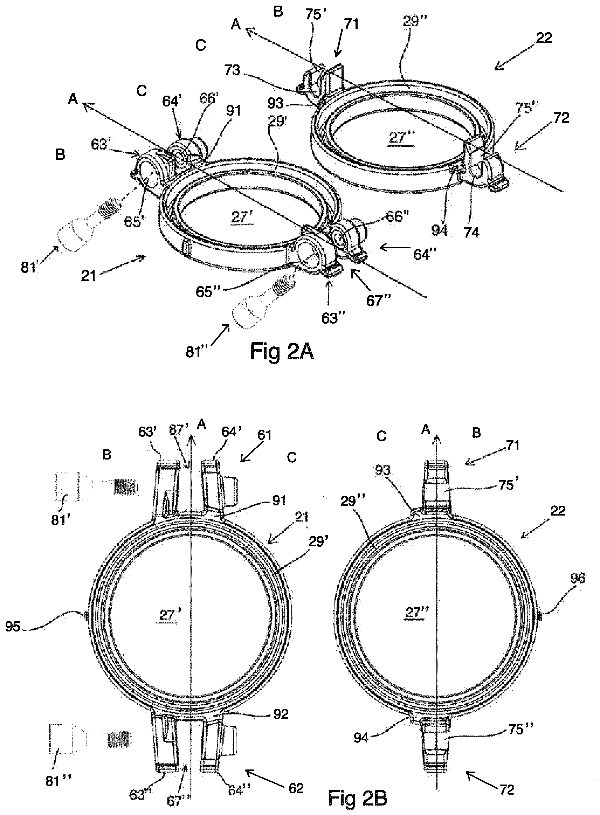

Each one of the first and second coupling elements 21, 22 are provided with a central opening 27', 27" (see Figs. 2A-B). The central openings 27', 27" may have any geometric outline such as circular, oval, quadratic or rectangular, extending in the same plane as the centerline A. In the embodiments shown in the figures herein, the central openings 27', 27" are circular. In embodiments wherein the central openings 27', 27" are circular the inner diameter of the openings 27', 27" may have a length of 10-50 mm, preferably 20-40 mm, more preferably 25-35 mm. The first and second coupling elements 21, 22 generally have the same outline as the central openings 27', 27". This means that if the central openings 27', 27" have a circular configuration, also the first and second coupling elements 21, 22 will have a generally circular outline. The first and second tubular connecting elements 31, 32 are removably fitted inside the circular openings 27', 27" of the first and second coupling elements 21, 22 and they can be freely rotated therein. The rotational capability of the tubular connecting elements 31, 32 in relation to the coupling elements 21, 22 is a great advantage during the implantation process since the surgeon may then adjust the fitting of the vascular coupling device 10 to the patient as well as the TAH in an optimal way.

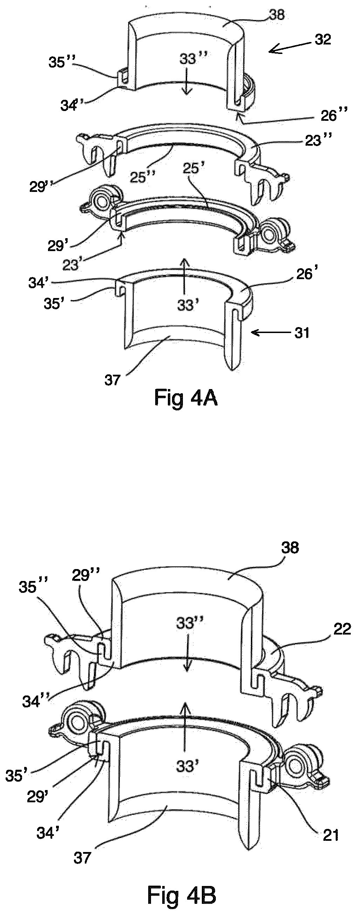

Each one of the coupling elements 21, 22 has an external side 23', 23" arranged opposite to a coupling side (see Figs. 1B and 4A). The external sides 23', 23" of the first and second coupling elements 21, 22 face the exterior of the vascular device 10, and coupling surfaces 25', 25" (see Fig. 4A) provided on the coupling sides are arranged to face each other when the first and second coupling elements 21, 22 of the vascular coupling device 10 are connected into a locked configuration.

The inner perimeters of the central openings 27', 27" are provided with grooves 29', 29" facing towards the coupling sides 25', 25" of the coupling elements 21, 22 (see Figs. 2A-B). Each one of the first and second coupling elements 21, 22 have a flat configuration of 2-8 mm, 3-7 mm in height.

The first and second coupling elements 21, 22 are advantageously made from a biocompatible material such as stainless steel, titanium and any other stiff biocompatible materials.

Each one of the central openings 27', 27" provided in the first and second coupling elements 21, 22 are arranged to receive a tubular connecting element 31, 32 therein. The tubular connecting elements 31, 32 generally have a tubular configuration with a geometric cross-section that corresponds to the geometric outline of the central openings 27', 27" of the coupling elements 21, 22. The tubular connecting elements 31, 32 are provided with a first open end 33', 33" and a second open end 37, 38 (see Fig. 4A-C). The tubular connecting elements 31, 32 are made from a bio-compatible material.

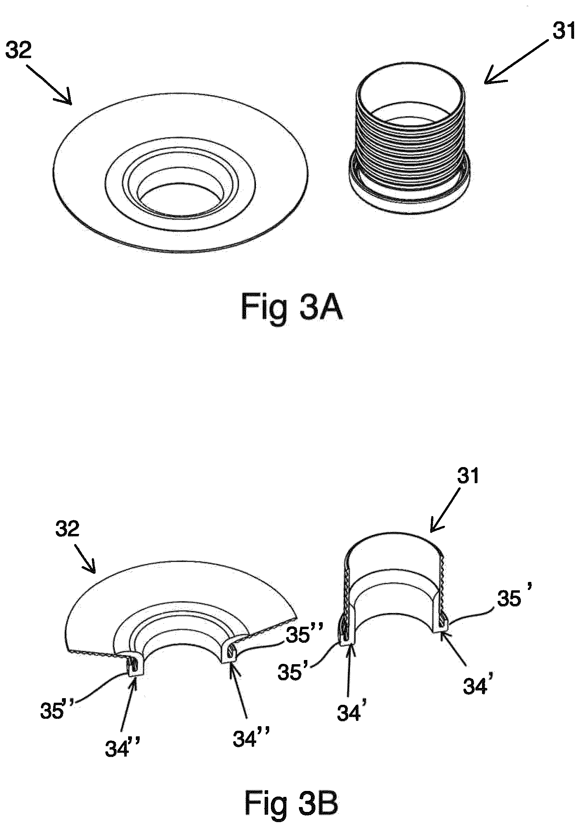

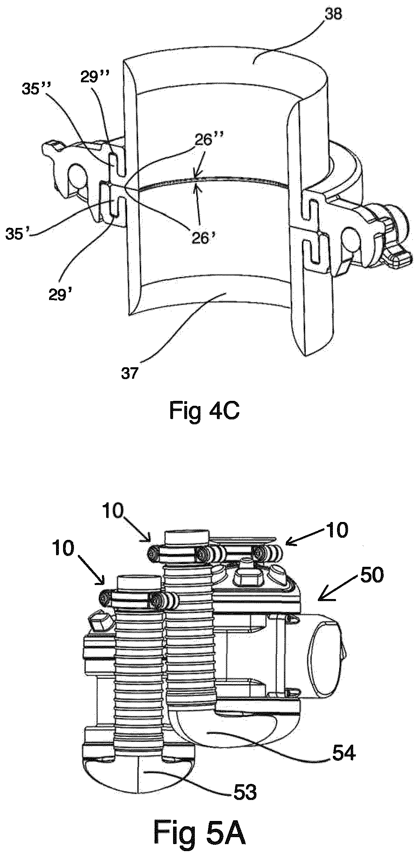

Advantageously, the first open ends 33', 33" of both the first and second tubular connecting elements 31, 32 are provided with a flange 34', 34" with an downwardly/upwardly turned lip 35', 35" (see Figs. 3B and 4A-C). The downwardly/upwardly turned lips 35', 35" are arranged to be received and rest in the grooves 29', 29" provided around the inner parameters of the central openings 27', 27" on the coupling sides of the first and second coupling elements 21, 22. When the first and second tubular connecting elements 31, 32 are fitted into the central openings 27', 27" of the first and second coupling elements 21, 22, the flanges 34', 34" will be flush with the surfaces of the coupling sides forming a coupling surface 26', 26" for connecting the first open ends 33', 33" of the first and second tubular connecting elements 21, 22 in a leak-proof manner (see fig. 4C).

The second open ends 37, 38 of each one of the first and second tubular connecting elements 31, 32 protrude through the central openings 27', 27" towards the exterior sides of each of the first and second coupling elements 21, 22 (see Figs. 1B-D and 4B-C).

In order to facilitate the fitting of the tubular connecting elements 31, 32 to the first and second coupling elements 21, 22, the material of the flanges 34', 34" is advantageously of a pliable and resilient character that immediately resumes its original shape after having been fitted into or removed from the central openings 27', 27". The material of the flanges 34', 34" and the tubular connecting elements 31, 32 may be chosen from materials of the group consisting of polyethylene, polyamide, polymethylmethacrylate, polytetrafluroethylene, polyurethane, and silicones such as dimethyl siloxane, polydimethylsiloxane, and decamethyl cyclopentasiloxane, or combinations thereof.

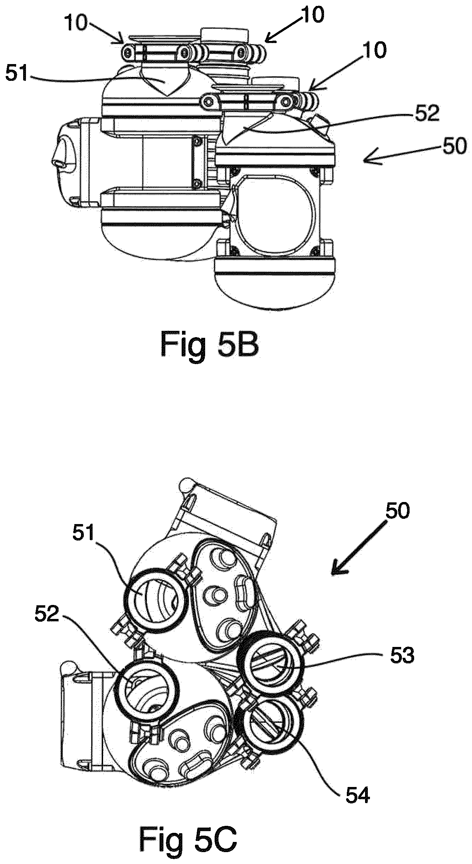

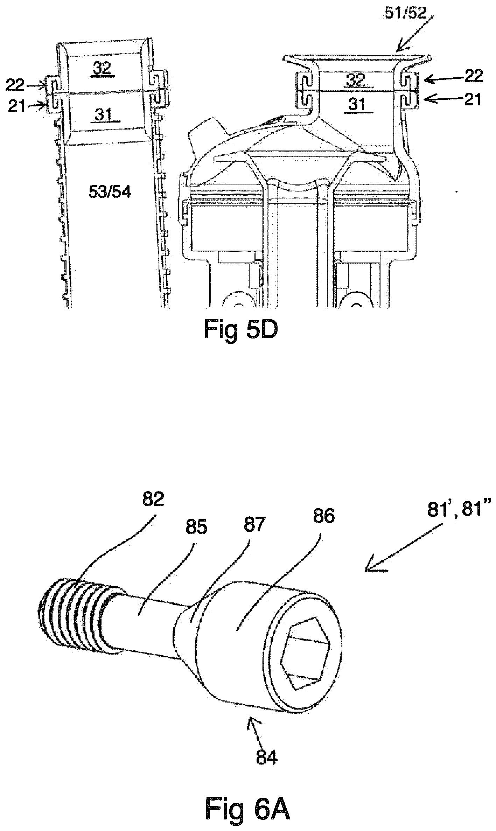

The second open end 37 of the first tubular connecting element 31 is arranged to be connected to either one of the inlet or the outlet channels of the TAH and is advantageously provided with a tubular element material having a length adapted to accommodate any strains that may appear in the connection between the vascular coupling device 10 and the TAH. The second end 37 may e.g. be reinforced with a metal wire to improve on the rigidity of the connection. For example, when the second end 37 of the first tubular connecting element 31 is connected to the inlet of the TAH, the second end 37 is advantageously shorter than when connected to the outlet channel (see e.g. Fig. 5D) and could be integrated into the material of the TAH.

The second open end 38 of the second tubular connecting element 32 is arranged to be connected to the vascular system of a patient and is advantageously provided with a vascular grafting material. The vascular grafting material must be blood compatible and is chosen from the group comprising any known commercially available vascular grafting materials such as e.g. polyethylene teraphthalate (Dacron), expanded polytetrafluoroethylene (ePTFE), Polyamide (nylon) and polyurethane. In one embodiment the vascular grafting material is polyethylene teraphthalate (Dacron).

However, it is also possible that the second open end 37 of the first tubular connecting element 31 is instead fitted with vascular grafting material, and the second open end 38 of the second tubular connecting element 32 is provided with the tubular element material to be connected to a TAH. Alternatively the second ends 37, 38 of both the first and second tubular connecting elements 31, 32 are provided with a vascular grafting material.

The first and second coupling elements 21, 22 are removably connected to each other into a locked configuration, or disconnected from each other into an unlocked configuration by means of a pair of locking structures 41, 42. The centerline A for the vascular coupling device 10 extends through the pair of locking structures 41, 42 which are arranged on the centerline A and opposite to each other on an outer perimeter of said vascular coupling device 10 (see Fig. 1D).

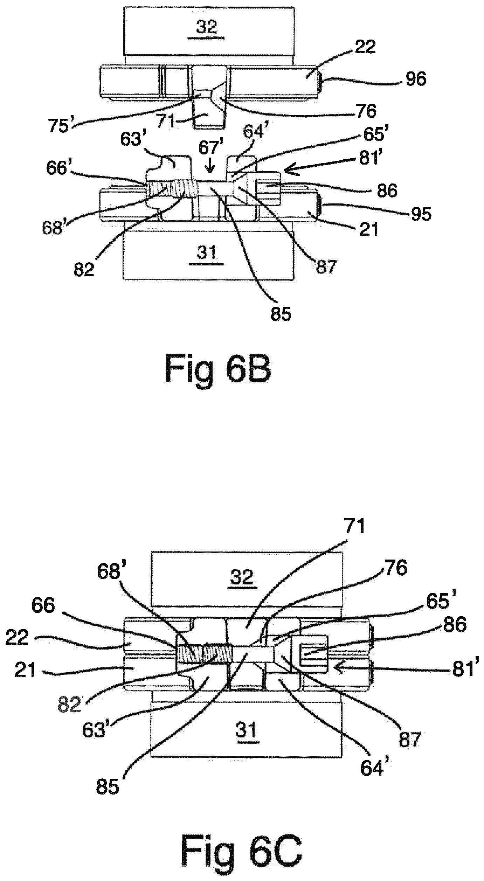

Each one of the locking structures 41, 42 comprises
- a female locking part 61, 62 arranged on the first coupling element 21; and
- a male locking part 71, 72 arranged on the second coupling element 22; and
- a locking element 81', 81" (see Fig. 6A) configured to join the female locking part 61, 62 with the male locking part 70, 71 into a locked configuration (see Figs. 1A-D and 2A-B). In the locked configuration the first and second coupling elements 21, 22 are brought into contact such that the docking surfaces 26', 26" on the flanges 34', 34" provided on the first open ends 33', 33" of the first and second tubular connecting elements 31, 32 meet and form a leak-proof conduit through which blood may pass (see Figs. 1D and 4C).

The vascular coupling device 10 comprises two locking structures 41, 42 (Fig. 1D) located opposite to each other on the centerline A of the vascular coupling device 10. Thus, each vascular coupling device 10 comprises a first and a second female locking part 61, 62 arranged opposite to each other on the centerline A of the first coupling element 21, a first and a second male coupling part 71, 72 arranged opposite to each other on the centerline A of the second coupling element 22, and a first and a second locking element 81', 81" configured to join the first female locking part 61 to the first male locking part 71, and the second female locking part 62 to the second male locking part 72 respectively (see Figs. 1C-D).

The first and second female locking parts 61, 62 are located on the centerline A and on the outer perimeter of the first coupling element 21. Each female locking part 61, 62 comprises a first lug 63', 63"and a second lug, 64', 64", with a gap 67', 67" located between the first lug 63', 63" and second lug 64', 64". As pointed out above, the pair of locking structures 41, 42 is arranged opposite to each other on the centerline A of the vascular coupling device 10, and consequently the first and second female locking parts 61, 62 are also arranged opposite to each other on the centerline A. Accordingly, the centerline A runs through the gaps 67', 67" of the first coupling element 21 placing said first lugs 63', 63"and second lugs 64', 64" on opposite sides B and C of said centerline A (see Fig. 2B).

The first lug 63', 63" has a first bore 65', 65" (located on side B of the centerline A), and the second lug 64', 64" has a second bore 66', 66" (located on side C of the centerline A), the second bore 66', 66" being provided with a screw thread on the inside wall 68', 68". The first bore 65', 65" has a first diameter which is larger than the diameter of the second bore 66', 66" (see Figs. 2A and 6B).

The first and second male locking parts 71, 72 are arranged on the centerline A and opposite to each other on the outer perimeter of the second coupling element 22 only. The male locking parts 71, 72 are configured to be received into the gaps 67', 67" between the first lugs 63', 63"and second lugs, 64', 64" of the female locking parts 61, 62 (see Figs. 1D and 2A-B).

The female and male locking parts 61, 62, 71, 72 respectively are connected together into a locked configuration by means of a locking element 81', 81". The locking element 81', 81" has a first end provided with an outer screw thread 83, a second end provided with a screw head 84, and a mid-section 85 arranged in between the first and second ends. As can be seen in figure 6A, the screw head 84 has two ends, a first end which has a cylindrical shape 86 and a second end which tapers from the cylindrical part into a cone shape 87 towards the mid-section 85 of the locking element 81', 81". The reason for this shape will be explained below.

The cylindrical part 86 on the first end of the screw head 84 has a diameter which matches the first diameter of the first bore 65', 65" of the female locking part 61, 62, and the screw threaded end 83 has a diameter that matches the diameter of the second bore 66', 66". The locking element 81', 81" is configured to be received into the first and second bores 65', 65", 66', 66" of the female locking part 61, 62 (see Fig. 6B).

The locking structure 41, 42 as described herein may show some similarities to a clevis joint but with some important differences which will be pointed out. The female locking part 61, 62 may have the shape and take the function of a clevis bracket. The male locking part 71, 72 may have the shape and take the function of a clevis eye, and the locking element 81 may have the shape and take the function of a clevis pin.

The locking elements 81', 81" are configured to enter with their first end 83', 83" (screw threaded end) through the first bore 65', 65" perpendicular to and from the same side B of the centerline A. Thereafter the first screw threaded ends 83', 83" cross the centerline A and are received into the second bores 66', 66" located on the opposite side C of the centerline A, while the second ends 84', 84" (i.e. the screw head) remain in the first bores 65', 65". This can be seen in figures 1D, 2A-B and 6B-D wherein it is shown that both of the screw heads 84', 84" of the locking elements 81', 81" are arranged in the first bores 65', 65" of both of the female locking parts 61, 62 and on the same side B of the centerline A. The mid-sections 85', 85" are arranged in the gaps 67', 67" between the first and second lugs 63', 63", 64', 64", reaching across the centerline A, and the screw threaded ends 84', 84" are arranged inside the second bores 66', 66" on the opposite side C of the centerline A.

Each one of the male locking parts 71, 72 comprises an eye (or bore) 73, 74 provided with an open cleft 75', 75". The open clefts 75', 75" have a width corresponding to the diameters of the mid-sections 85', 85" of the locking elements 81', 81" with their openings facing towards the coupling side 25" of the second coupling element 22 (see Figs. 2A and 4A-C).

The mid-sections 85', 85" of the locking elements 81', 81" are configured to be received into the open clefts 75', 75" provided on the eyes 73, 74 of the male locking parts 71, 72 when the male locking parts 71 72 are arranged in the gaps 67', 67" between the first and second lugs 63', 63", 64', 64" of the female locking parts 61, 62 (see Figs. 6B-C).

Each eye 73, 74 is provided with a first and second recess 76, 77 arranged on one side of the eye 73, 74 only. As seen in figure 1B both of the first and second recesses 76, 77 are located on side B of the centerline A. The first and second recesses 76, 77 are configured to receive the cone shaped ends 87', 87" of the locking element screw heads 84', 84". Both of the first and second recesses 76, 77 are arranged on the eyes 73, 74 such that they face in the same direction and away from the centerline A.

One advantageous feature of the vascular coupling device 10 described herein, is that the operator can be assured that the vascular coupling device 10 always is fitted in a correct way. This is extremely important since an incorrect assembly of the vascular coupling device 10 can have devastating consequences. In order to avoid problems of accidental incorrect fitting, the vascular coupling device 10 disclosed herein is provided with a fail-safe arrangement. This fail-safe arrangement comprises first and second cut-in portions 91, 92 arranged on the first coupling element 21 that cooperate with first and second projecting elements 93, 94 arranged on the second coupling element 22.

The first and second cut-in portions 91, 92 are arranged on an outer perimeter of the first coupling element 21. In the figures 2A-B the cut-in portions 91, 92 form a part of the female locking part 61, 62 on the first coupling element 21, but the exact location on the first coupling element 21 is not important as long as both the first and the second cut-in portions 91, 92 are located on the same side of the centerline A, i.e. either both are located on side B or on side C. In the embodiments show in the figures, the first and second cut-in portions 91, 92 are arranged on the coupling side of the second lug 64', 64", i.e. on side C of the centerline A (see Fig. 1D and 2A-B).

The first and second projecting elements 93, 94 are arranged on an outer perimeter of the second coupling element 22. In the figures 2A-B the projecting elements 93, 94 are located next to the male locking part 71, 72 but the exact location on the outer perimeter is not important as long as both the first and the second projecting elements 93, 94 are located on the same side of the centerline A and arranged to cooperate with the first and second cut-in portions 91, 92 on the first coupling element 21, when the vascular coupling device 10 is in the locked configuration. In the embodiments shown in the figures, the first and second projecting elements 93, 94 are arranged on the C side of the centerline A, and opposite to the first and second recesses 76, 77 provided around the eyes 73, 74 (see Fig. 1B).

In order to function as a fail-safe arrangement, an erroneous assembly of the coupling elements 21, 22 must be prevented. The first and second projecting elements 93, 94 are configured to be received into the first and second cut-in portions 91, 92 when the vascular coupling device 10 is in a locked configuration. Consequently the first and second cut-in portions 91, 92 on the first coupling element 21 must be located such that they will mate with the first and second blocking elements 93, 94 on the second blocking element 22 when the vascular device is in the locked configuration.

This mirror image configuration of the fail-safe arrangement prevents any incorrect assembly of the vascular coupling device 10. It is therefore impossible to assemble the first and second locking structures 41, 42 in an incorrect manner since trying to fit the first and second locking elements 21, 22 together when one of them is turned the wrong way is prevented by the projecting elements 93, 94 not being able to be fitted into the cut-in portions 91, 92. When the cut-in portions 91, 92 are arranged on the second lugs 64', 64" as seen in the figures 2A-B any attempt to assemble an incorrectly positioned coupling element 21, 22, e.g. the first coupling element 21 to the second coupling element 22 will be prevented when the projecting elements 93, 94 arranged on the second coupling element 22 end up opposite to the first lugs 63', 63" on the first coupling element 21 (i.e. the lugs without the cut-in portions). It is therefore impossible to assemble the first and second coupling elements 21, 22 together the wrong way.

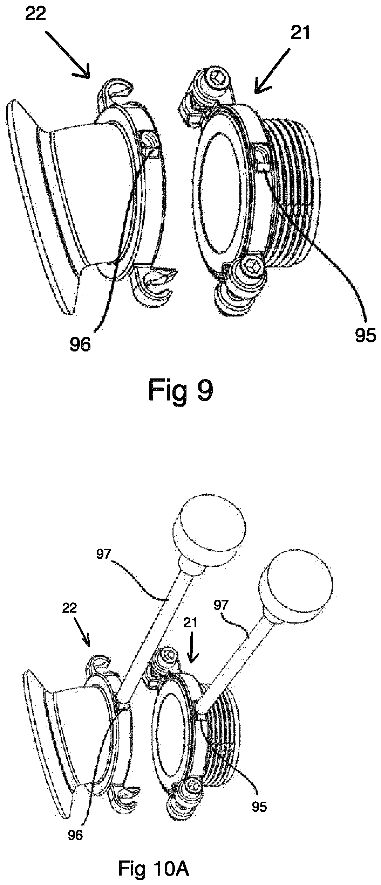

In order to facilitate the handling of the vascular coupling device 10 during implantation of the vascular coupling device to the vascular system of the patient, the vascular coupling device 10 is provided with first and second receiving means 95, 96, advantageously provided with inner screw threads (see Fig. 9). The first receiving means 95 is located on the first coupling element 21 and the second receiving means 96 is located on the second coupling element 22, both first and second receiving means 95, 96 are located on the same side of the center line A. As can be seen in figures 1C, 2B and 9A, the first and second receiving means 95, 96 are located on the B side of the centerline A. However, the first and second receiving means 95, 96 may of course be located on the opposite side C instead.

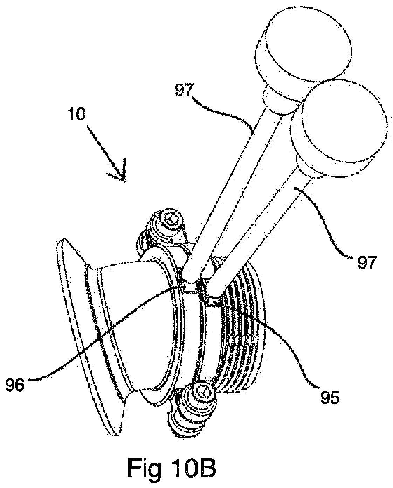

Each one of the first and second receiving means 95, 96 are configured to receive a holding means 97 (see Figs. 10A and 10B), configured to be connected thereto and used for holding and manipulating the vascular coupling device 10 during the implantation process. The holding means 97 is advantageously provided with an outer screw thread adapted to mesh with the inner screw thread provided in the first and second receiving means 95, 96.

A method of fitting a TAH to a vascular system of a patient by means of the vascular coupling device 10 as disclosed herein will now be described in more detail. An advantageous feature of the vascular coupling device 10 is that the operator is able to connect and/or disconnect the vascular system of a patient to a TAH or other type of heart pump device quickly without having to perform many time consuming steps. It is an advantage that most of the parts of the vascular coupling device 10 can be prepared and fitted beforehand in order for the connecting/disconnecting operation to take a minimum amount of time.

The first and second tubular connecting elements 31, 32 are fitted into the first and second coupling elements 21, 22 respectively such that the downwardly/upwardly turned lips 35', 35" are resting inside the grooves 29', 29" on the inner parameters of the central openings 27', 27", and the second open ends 37, 38 are protruding through the central openings 27', 27" towards the external sides of each coupling element 21, 22 (see Figs. 1B and 4B). This step is advantageously performed before surgery has started.

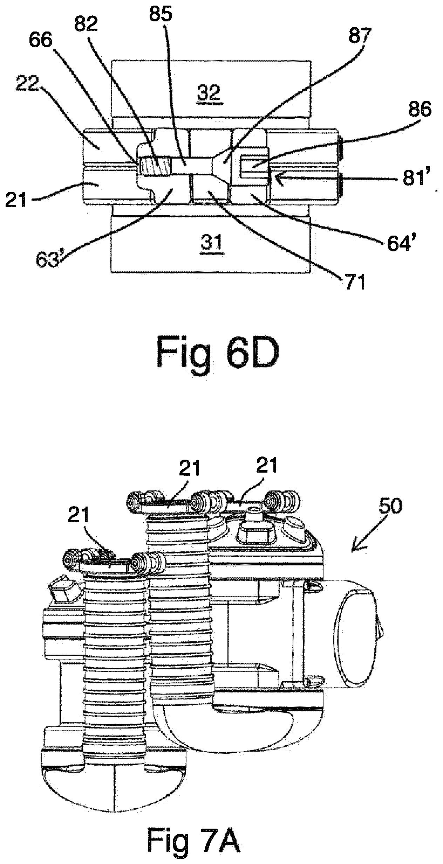

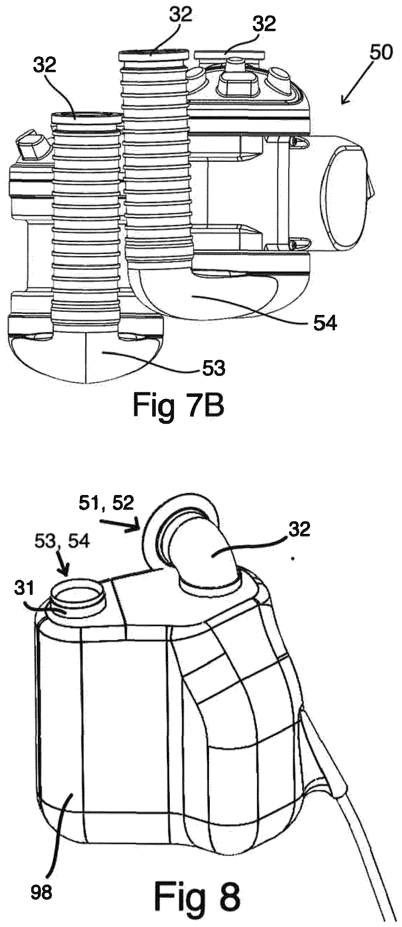

The second open end 37 of the first tubular connecting element 31 protruding from the central opening 27' of the first coupling element 21 is advantageously provided with a rigid tubular element reinforced with a metal wire which is used for connecting the vascular coupling device 10 to an inlet 51, 52 or outlet 53, 54 of a TAH 50. This connection may be prepared in advance of the surgery such that the rigid tubular element reinforced with a metal wire is connected to the inlet/outlet of the TAH and the first end 33' of the first tubular connecting element 31 provided with the flange 34' and the downwardly turned lip 35' is mounted in the groove 27' on the first coupling element 21. Advantageously it is the first coupling element 21 comprising the female locking parts 61, 62 that are arranged on the inlets 51, 52 / outlets 53, 54 of the TAH 50 in advance (see Fig. 7A). However, it is possible that under some circumstances also the second coupling element 22 comprising the male coupling parts 71, 72 may be fitted to the inlet 51, 52 / outlet 53, 54 of the TAH 50 already at the start of the procedure. As a TAH 50 normally has two inlet channels 51, 52 and two outlet channels 53, 54. All four channels 51, 52, 53, 54 may be fitted with at least a tubular connecting element 31 (see Fig. 7B) but optionally also with the coupling element 21, 22 of the vascular coupling device 10 before surgery has started (see Fig. 7A).

In order to transfer the vascular coupling device 10 into a locked configuration, both of the locking elements 81', 81" are entered (from the same side, i.e. side B of the center line A) through the first bores 65', 65" of the female locking parts 61, 62 such that the outer screw threads 82', 82" of the locking elements 81', 81" are received into and partly joined with the inner screw threads 68', 68" provided inside the second bores 66', 66". The screw heads 83', 83" of the locking elements 81', 81" are received into and rest inside the first bores 65', 65". This step may also be prepared before starting surgery (see Figs. 1B, 4B and 6B).

When the chest of the patient has been opened and the biological heart removed, the vascular grafting material provided on the on the second end 38 of the second tubular connecting element 32 may be connected to the arteries or veins, alternatively to the remaining parts of the natural atria of the natural heart. Advantageously, the second tubular connecting element 32 has already been fitted into the second coupling element 22 such that the upwardly turned lip 35" is resting inside the groove 29" along the inner parameter of the central opening 27", and the second open end 38 with the vascular grafting material is protruding through the central opening 27" towards the external side of the second coupling element 22. Consequently the male locking parts 71, 72 are arranged on the part of the vascular device 10 connected to the arteries/veins alternatively the remaining parts of atria.

In order to connect the vascular device 10 into a locked configuration, the first and second male locking parts 71, 72 are placed into the gaps 67', 67" arranged between the first and second lugs 63', 63", 64', 64" of the female locking parts 61, 62 such that the mid-sections 85', 85" of the locking elements 81', 81" are received into the open clefts 75', 75" of the eyes 73, 74 on the male locking elements 71, 72 (see Figs. 1C, 4C and 6C). This will not be possible unless both of the first and second coupling elements 21, 22 are turned in the right direction. If one of the coupling elements 21, 22 are turned the wrong way, the first and second coupling elements 21, 22 will not assemble due to the fail-safe arrangement.

The rotational capability of the tubular connecting elements 31, 32 in relation to the coupling elements 21, 22 is a great advantage during the implantation process since if there is some strain between the vascular coupling device 10 and the veins/arteries of the patient, or between the vascular device and the TAH, the surgeon may then rotate one or both of the tubular connecting elements 31, 32 to optimize the fitting and release the strain or stress.

Thereafter, the ends of the locking element 81', 81" provided with screw threads 82', 82" are screwed all the way into the second bores 66, 66', thereby causing the cone shaped part 87', 87" of the screw heads 84, 84' to be received into the first and second recesses 76, 77 provided on the eyes 73, 74 of the male locking parts 71, 72 (see Fig. 6D). When the cone shaped part 87', 87" of the screw heads 84', 84" enter the first and second recesses 76, 77 of the eyes 73, 74, the first and second coupling elements 21, 22 are pulled together further into a firm locking configuration. Simultaneously the coupling surfaces 26', 26" on the flanges 34', 34" provided on the first open ends 33', 33" of the first and second tubular connecting elements 31, 32 meet and form a tight and leak-proof conduit through which blood may pass (see Fig. 4C).

Advantageously an encasing sac 98 is attached to the external surfaces 23' of the coupling device 10 facing away from the TAH when the vascular coupling device 10 is connected to the inlet channels and outlet channels 53, 54 of the TAH (see figure 8). The encasing sac 98 receives and encloses the artificial heart pumps 50 to protect the pump from tissue ingrowth.

## Claims

1. A vascular coupling device (10), comprising
- a first and a second coupling element (21, 22) wherein each one of said first and second coupling elements (21, 22) has an external surface (23', 23") facing an external side, a coupling surface (25', 25") facing a coupling side, and a central opening (27', 27"); and
- a first and a second tubular connecting element (31, 32), wherein each one of said first and second tubular elements (31, 32) has a first open end (33', 33") and a second open end (37, 38), said first open ends (33', 33") being provided with flanges (34', 34");
each one of said first and second tubular connecting elements (31, 32) is arranged in a corresponding central opening (27', 27") of the first and second coupling elements (21, 22) respectively, and with said second open ends (37, 38) protruding through said central openings (27', 27") on said external side of each of said first and second coupling elements (21, 22);
said first and second coupling elements (21, 22) being removably connected to each other into a locked configuration, or disconnected from each other into an unlocked configuration by means of a first and second locking structure (41, 42), said first and second locking structures (41, 42) being arranged on a centerline A of the vascular coupling device (10), and opposite to each other on an outer perimeter of said vascular coupling device (10),
**characterized in that** said vascular device further comprises a fail-safe arrangement comprising first and second cut-in portions (91, 92) arranged on said first coupling element (21) configured to receive first and second projecting elements (93, 94) arranged on said second coupling element (22), said first and second cut-in portions (91, 92) and first and second projecting element (93, 94) being arranged on same side of said centerline A, thereby preventing erroneous connection of said first and second coupling elements (21, 22) to each other.

2. The vascular coupling device according to claim 1, wherein inner perimeters of said central openings (27', 27") are provided with grooves (29', 29") facing said coupling sides.

3. The vascular coupling device according to claims 1 and 2, wherein said flanges (34', 34") on said first open ends (33', 33") of each of said first and second tubular connecting elements (31, 32) are provided with downwardly/upwardly turned lips (35', 35").

4. The vascular coupling device according to claim 3, wherein said downwardly/upwardly turned lips (35', 35") provided on said flanges (34', 34") are resting in said grooves (29', 29") on the inner diameters of the central openings (27', 27").

5. The vascular coupling device according to any one of the preceding claims, wherein each one of said first and second locking structures (41, 42) comprises
- a female locking part (61, 62) arranged on said first coupling element (21); and
- a male locking part (71, 72) arranged on said second coupling element (22); and
- a locking element (81', 81") configured to join said female locking part (61, 62) with said male locking part (71, 72) into a locked configuration, wherein said coupling surfaces (25', 25") of said first and second coupling elements (21, 22) are brought into contact such that said first open ends (33', 33") of said first and second tubular connecting elements (31, 32) are brought into a sealing connection

6. The vascular coupling device according to claim 5, wherein
- each one of said female locking parts (61, 62) comprises a first and a second lug (63', 63", 64', 64") having a gap (67', 67") therebetween, said first and second lugs (63', 63", 64', 64") being arranged on opposite sides of said centerline A, said first lug (63', 63") having a first bore (65', 65"), and said second lug (64', 64") having a second bore (66', 66") provided with an inner screw thread; and
- each one of said male locking parts (71, 72) is arranged on the centerline A and is configured to be received into said gap (67', 67") between said first and second lugs (63', 63", 64', 64") of said female locking parts (61, 62); and
- each one of said locking elements (81', 81") has a first end provided with an outer screw thread (82',82"), a second end provided with a cone shaped head (87', 87"), and a mid-section (85', 85") arranged between said first and second ends, said locking elements (81', 81") are configured to be received into said first and second bores (65', 65", 66', 66") of said female locking part (61, 62).

7. The vascular coupling device according to claim 6, wherein both of said locking elements (81', 81") are configured to be entered into said first bores (65', 65") from a first side and perpendicular to said centerline A, cross said centerline A and thereafter be received into said second bores (66', 66") provided with inner screw threads and arranged on a second side of said centerline A opposite to said first side.

8. The vascular coupling device according to any one of the preceding claims, wherein each one of said male locking parts (71, 72) comprises an eye (73, 74) provided with an open cleft (75', 75") facing said coupling side of said second coupling element (22).

9. The vascular coupling device according to claim 8, wherein said mid-sections (85', 85") of said locking elements (81', 81") are configured to be received into said open clefts (75', 75") provided on said eyes (73, 74) of said male locking parts (71, 72) when said male locking parts (71, 72) are arranged in said gaps (67', 67") between said first and second lugs (63', 63", 64', 64") of said female locking parts (61, 62).

10. The vascular coupling device according to claims 8 and 9, wherein first and second recesses (76, 77) are arranged around inner perimeters of said eyes (73, 74) and on same side of said centerline A of said male locking parts (71, 72), said first and second recesses (76, 77) being configured to receive said cone-shaped heads (87', 87") of said locking elements (81', 81").

11. The vascular coupling device according to claim 10, wherein said vascular (10) device is in a locked configuration when said outer screw threads (82', 82") of said locking elements (81', 81") are received into and joined with said inner screw threads provided in said second bores (66', 66") of said female locking parts (61, 62), and said cone-shaped heads (87', 87") of said locking elements (81', 81") are received into said recesses (76, 77) provided on said eyes (73, 74) arranged on said male locking parts (71, 72).

12. The vascular coupling device according to any one of the preceding claims, wherein said first and a second projecting element (93, 94) are arranged on an outer perimeter of said second coupling element (22),

13. The vascular coupling device according to any one of claims 10-11 or claim 12 when dependent on claim 10 or 11, wherein said first and second projecting elements (93, 94) and said recesses (76, 77) are arranged on opposite sides of said centerline A o said second coupling element (22).

14. The vascular coupling device according to any one of the preceding claims, wherein said first and a second cut-in portions (91, 92,) are arranged on an outer perimeter of said first coupling element (21),

15. The vascular coupling device according to any one of the preceding claims wherein an encasing sac (98) is attached to the external surface (23', 23") of the coupling device and receives and encloses the artificial heart pump 50 to protect the pump from tissue ingrowth.

16. The vascular coupling device according to any one of the preceding claims wherein a first and second receiving means (95, 96) are arranged on the first and second coupling elements (21, 22) respectively, both first and second receiving means (95, 96) being arranged on the same side of the center line A and are adapted to facilitate manipulation of said vascular coupling device (10) during implantation.

17. A total artificial heart (TAH) (50) comprising a first and a second inlet channel (51, 52) and a first and a second outlet channel (53, 54), wherein one or more of the first and second inlet and outlet channels (51, 52, 53, 54) are connected to a vascular coupling device (10) according to any one of claims 1-16.

## Patentansprüche

1. Gefäßkopplungsvorrichtung (10), umfassend
- ein erstes und ein zweites Kopplungselement (21, 22), wobei jedes der ersten und zweiten Kopplungselemente (21, 22) eine Außenoberfläche (23', 23"), die einer Außenseite zugewandt ist, eine Kopplungsoberfläche (25', 25"), die einer Kopplungsseite zugewandt ist, und eine zentrale Öffnung (27', 27") aufweist; und
- ein erstes und ein zweites röhrenförmiges Verbindungselement (31, 32), wobei jedes der ersten und zweiten röhrenförmigen Elemente (31, 32) ein erstes offenes Ende (33', 33") und ein zweites offenes Ende (37, 38) aufweist, wobei die ersten offenen Enden (33', 33") mit Flanschen (34', 34") versehen sind;
jedes der ersten und zweiten röhrenförmigen Verbindungselemente (31, 32) in einer entsprechenden zentralen Öffnung (27', 27") des ersten beziehungsweise des zweiten Kopplungselements (21, 22) und mit den zweiten offenen Enden (37, 38), die durch die zentralen Öffnungen (27', 27") auf der Außenseite jedes der ersten und zweiten Kopplungselemente (21, 22) vorstehen, angeordnet ist;
wobei die ersten und zweiten Kopplungselemente (21, 22) lösbar in einer verriegelten Konfiguration miteinander verbunden oder in einer entriegelten Konfiguration voneinander getrennt sind, mittels einer ersten und zweiten Verriegelungsstruktur (41, 42), wobei die ersten und zweiten Verriegelungsstrukturen (41, 42) auf einer Mittellinie A der Gefäßkopplungsvorrichtung (10) und einander gegenüberliegend an einem äußeren Umfang der Gefäßkopplungsvorrichtung (10) angeordnet sind,
**dadurch gekennzeichnet, dass** die Gefäßvorrichtung ferner eine ausfallsichere Anordnung umfasst, umfassend erste und zweite eingeschnittene Abschnitte (91, 92), die auf dem ersten Kopplungselement (21) angeordnet und konfiguriert sind, um erste und zweite vorstehende Elemente (93, 94) aufzunehmen, die auf dem zweiten Kopplungselement (22) angeordnet sind, wobei die ersten und zweiten eingeschnittenen Abschnitte (91, 92) und das erste und zweite vorstehende Element (93, 94) auf der gleichen Seite der Mittellinie A angeordnet sind, wobei dadurch eine fehlerhafte Verbindung der ersten und zweiten Kopplungselemente (21, 22) miteinander verhindert wird.

2. Gefäßkopplungsvorrichtung nach Anspruch 1, wobei innere Umfänge der zentralen Öffnungen (27', 27") mit Rillen (29', 29") versehen sind, die den Kopplungsseiten zugewandt sind.

3. Gefäßkopplungsvorrichtung nach Anspruch 1 und 2, wobei die Flansche (34', 34") an den ersten offenen Enden (33', 33") von jedem der ersten und zweiten röhrenförmigen Verbindungselemente (31, 32) mit nach unten/oben gedrehten Lippen (35', 35") versehen sind.

4. Gefäßkopplungsvorrichtung nach Anspruch 3, wobei die an den Flanschen (34', 34") vorgesehenen nach unten/oben gedrehten Lippen (35', 35") in den Rillen (29', 29") an den inneren Durchmessern der zentralen Öffnungen (27', 27") ruhen.

5. Gefäßkopplungsvorrichtung nach einem der vorstehenden Ansprüche, wobei jede der ersten und zweiten Verriegelungsstrukturen (41, 42) umfasst
- ein weibliches Verriegelungsteil (61, 62), das an dem ersten Kopplungselement (21) angeordnet ist; und
- ein männliches Verriegelungsteil (71, 72), das an dem zweiten Kopplungselement (22) angeordnet ist; und
- ein Verriegelungselement (81', 81"), das konfiguriert ist, um das weibliche Verriegelungsteil (61, 62) mit dem männlichen Verriegelungsteil (71, 72) in eine verriegelte Konfiguration zusammenzuführen, wobei die Kopplungsoberflächen (25', 25") der ersten und zweiten Kopplungselemente (21, 22) derart in Berührung gebracht werden, dass die ersten offenen Enden (33', 33") der ersten und zweiten rohrförmigen Verbindungselemente (31, 32) in eine abdichtende Verbindung gebracht werden

6. Gefäßkopplungsvorrichtung nach Anspruch 5, wobei
- jedes der weiblichen Verriegelungsteile (61, 62) einen ersten und einen zweiten Ansatz (63', 63", 64', 64") umfasst, der eine Lücke (67', 67") dazwischen aufweist, wobei die ersten und zweiten Ansätze (63', 63", 64', 64") auf gegenüberliegenden Seiten der Mittellinie A angeordnet sind, wobei der erste Ansatz (63', 63") eine erste Bohrung (65', 65") aufweist und der zweite Ansatz (64', 64") eine zweite Bohrung (66', 66") aufweist, die mit einem inneren Schraubengewinde versehen ist; und
- jedes der männlichen Verriegelungsteile (71, 72) auf der Mittellinie A angeordnet und konfiguriert ist, um in die Lücke (67', 67") zwischen den ersten und zweiten Ansätzen (63', 63", 64', 64") der weiblichen Verriegelungsteile (61, 62) aufgenommen zu werden; und
- jedes der Verriegelungselemente (81', 81") ein erstes Ende, das mit einem äußeren Schraubengewinde (82', 82") versehen ist, ein zweites Ende, das mit einem kegelförmigen Kopf (87', 87") versehen ist, und einen Mittelbereich (85', 85") aufweist, der zwischen den ersten und zweiten Enden angeordnet ist, wobei die Verriegelungselemente (81', 81") konfiguriert sind, um in den ersten und zweiten Bohrungen (65', 65", 66', 66") des weiblichen Verriegelungsteils (61, 62) aufgenommen zu werden.

7. Gefäßkopplungsvorrichtung nach Anspruch 6, wobei beide Verriegelungselemente (81', 81") konfiguriert sind, um in die ersten Bohrungen (65', 65") von einer ersten Seite und senkrecht zu der Mittellinie A eingeführt zu werden, die Mittellinie A zu queren und danach in den zweiten Bohrungen (66', 66") aufgenommen zu werden, die mit inneren Schraubengewinden versehen und auf einer zweiten Seite der Mittellinie A gegenüber der ersten Seite angeordnet sind.

8. Gefäßkopplungsvorrichtung nach einem der vorstehenden Ansprüche, wobei jedes der männlichen Verriegelungsteile (71, 72) eine Öse (73, 74) umfasst, die mit einem offenen Spalt (75', 75") versehen ist, der der Kopplungsseite des zweiten Kopplungselements (22) zugewandt ist.

9. Gefäßkopplungsvorrichtung nach Anspruch 8, wobei die Mittelbereiche (85', 85") der Verriegelungselemente (81', 81") konfiguriert sind, um in den offenen Spalten (75', 75") aufgenommen zu werden, die an den Ösen (73, 74) der männlichen Verriegelungsteile (71, 72) bereitgestellt sind, wenn die männlichen Verriegelungsteile (71, 72) in den Lücken (67', 67") zwischen den ersten und zweiten Ansätzen (63', 63", 64', 64") der weiblichen Verriegelungsteile (61, 62) angeordnet sind.

10. Gefäßkopplungsvorrichtung nach den Ansprüchen 8 und 9, wobei erste und zweite Aussparungen (76, 77) um innere Umfänge der Ösen (73, 74) herum und auf der gleichen Seite der Mittellinie A der männlichen Verriegelungsteile (71, 72) angeordnet sind, wobei die ersten und zweiten Aussparungen (76, 77) konfiguriert sind, um die kegelförmigen Köpfe (87', 87") der Verriegelungselemente (81', 81") aufzunehmen.

11. Gefäßkopplungsvorrichtung nach Anspruch 10, wobei sich die Gefäßvorrichtung (10) in einer verriegelten Konfiguration befindet, wenn die äußeren Schraubengewinde (82', 82") der Verriegelungselemente (81', 81") darin aufgenommen und mit den inneren Schraubengewinden zusammengeführt werden, die in den zweiten Bohrungen (66', 66") der weiblichen Verriegelungsteile (61, 62) bereitgestellt sind, und die kegelförmigen Köpfe (87', 87") der Verriegelungselemente (81', 81") in den Aussparungen (76, 77) aufgenommen werden, die an den Ösen (73, 74) bereitgestellt sind, die an den männlichen Verriegelungsteilen (71, 72) angeordnet sind.

12. Gefäßkopplungsvorrichtung nach einem der vorstehenden Ansprüche, wobei das erste und ein zweites vorstehendes Element (93, 94) an einem äußeren Umfang des zweiten Kopplungselements (22) angeordnet sind.

13. Gefäßkopplungsvorrichtung nach einem der Ansprüche 10 bis 11 oder Anspruch 12, wenn von Anspruch 10 oder 11 abhängig, wobei die ersten und zweiten vorstehenden Elemente (93, 94) und die Aussparungen (76, 77) auf gegenüberliegenden Seiten der Mittellinie A oder des zweiten Kopplungselements (22) angeordnet sind.

14. Gefäßkopplungsvorrichtung nach einem der vorstehenden Ansprüche, wobei der erste und ein zweiter eingeschnittener Abschnitt (91, 92) an einem äußeren Umfang des ersten Kopplungselements (21) angeordnet sind.

15. Gefäßkopplungsvorrichtung nach einem der vorstehenden Ansprüche, wobei ein umhüllender Beutel (98) an der Außenoberfläche (23', 23") der Kopplungsvorrichtung befestigt ist und die künstliche Herzpumpe 50 aufnimmt und umschließt, um die Pumpe vor Gewebeeinwuchs zu schützen.

16. Gefäßkopplungsvorrichtung nach einem der vorstehenden Ansprüche, wobei erste und zweite Aufnahmemittel (95, 96) an dem ersten beziehungsweise dem zweiten Kopplungselement (21, 22) angeordnet sind, wobei sowohl erste als auch zweite Aufnahmemittel (95, 96) auf der gleichen Seite der Mittellinie A angeordnet und geeignet sind, eine Manipulation der Gefäßkopplungsvorrichtung (10) während der Implantation zu ermöglichen.

17. Künstlicher Totalersatz des Herzens (TAH) (50), umfassend einen ersten und einen zweiten Einlasskanal (51, 52) und einen ersten und einen zweiten Auslasskanal (53, 54), wobei einer oder mehrere der ersten und zweiten Einlass- und Auslasskanäle (51,52,53,54) mit einer Gefäßkopplungsvorrichtung (10) nach einem der Ansprüche 1 bis 16 verbunden sind.

## Revendications

1. Dispositif de raccord vasculaire (10), comprenant
- un premier et un second élément de raccord (21, 22), chacun desdits premier et second éléments de raccord (21, 22) ayant une surface externe (23', 23") faisant face à un côté externe, une surface de raccord (25', 25") faisant face à un côté de raccord, et une ouverture centrale (27', 27") ; et
- un premier et un second élément de raccordement tubulaire (31, 32), chacun desdits premier et second éléments tubulaires (31, 32) ayant une première extrémité ouverte (33', 33") et une seconde extrémité ouverte (37, 38), lesdites premières extrémités ouvertes (33', 33") étant pourvues de brides (34', 34 ») ;
chacun desdits premier et second éléments de raccordement tubulaires (31, 32) est agencé dans une ouverture centrale correspondante (27', 27") des premier et seconds éléments de raccord (21, 22) respectivement, et avec lesdites secondes extrémités ouvertes (37, 38) faisant saillie à travers lesdites ouvertures centrales (27', 27") sur ledit côté externe de chacun desdits premier et seconds éléments de raccord (21, 22) ;
lesdits premier et seconds éléments de raccordement (21, 22) étant raccordés de manière amovible l'un à l'autre dans une configuration verrouillée, ou sans être raccordés l'un à l'autre dans une configuration déverrouillée au moyen d'une première et d'une seconde structure de verrouillage (41, 42), lesdits première et seconde structures de verrouillage (41, 42) étant agencées sur une ligne centrale A du dispositif de raccord vasculaire (10), et opposées l'une à l'autre sur un périmètre extérieur dudit dispositif de raccord vasculaire (10),
**caractérisé en ce que** ledit dispositif vasculaire comprend en outre un agencement à sécurité intégrée comprenant des première et seconde parties découpées (91, 92) agencées sur ledit premier élément de raccord (21) conçues pour recevoir des premier et second éléments saillants (93, 94) agencés sur ledit second élément de raccord (22), lesdites première et seconde parties découpées (91, 92) et les premier et second éléments saillants (93, 94) étant agencés du même côté de ladite ligne centrale A, empêchant ainsi un raccord erroné desdits premier et second éléments de raccord (21, 22) l'un à l'autre.

2. Dispositif de raccord vasculaire selon la revendication 1, dans lequel les périmètres intérieurs desdites ouvertures centrales (27', 27") sont pourvus de rainures (29', 29") faisant face auxdits côtés de raccord.

3. Dispositif de raccord vasculaire selon les revendications 1 et 2, dans lequel lesdites brides (34', 34") sur lesdites premières extrémités ouvertes (33', 33") de chacun desdits premier et seconds éléments de raccordement tubulaires (31, 32) sont prévues avec des lèvres tournées vers le bas/vers le haut (35', 35").

4. Dispositif de raccord vasculaire selon la revendication 3, dans lequel lesdites lèvres tournées vers le bas/vers le haut (35', 35") prévues sur lesdites brides (34', 34") reposent dans lesdites rainures (29', 29") sur les diamètres intérieurs des ouvertures centrales (27', 27").

5. Dispositif de raccord vasculaire selon l'une quelconque des revendications précédentes, dans lequel chacune desdites première et seconde structures de verrouillage (41, 42) comprend
- une pièce de verrouillage femelle (61, 62) agencée sur ledit premier élément de raccord (21) ; et
- une pièce de verrouillage mâle (71, 72) agencée sur ledit second élément de raccord (22) ; et
- un élément de verrouillage (81', 81 ") conçu pour joindre ladite pièce de verrouillage femelle (61, 62) avec ladite pièce de verrouillage mâle (71, 72) dans une configuration verrouillée, dans lequel lesdites surfaces de raccord (25', 25") desdits premier et second éléments de raccord (21, 22) sont amenées en contact de telle sorte que lesdites premières extrémités ouvertes (33', 33") desdits premier et second éléments de raccordement tubulaires (31, 32) sont amenées dans un raccord étanche

6. Dispositif de raccord vasculaire selon la revendication 5, dans lequel
- chacune desdites pièces de verrouillage femelles (61, 62) comprend une première et une seconde patte (63', 63", 64', 64") ayant un espace (67', 67") entre elles, lesdites première et seconde patte (63', 63", 64', 64") étant disposées sur des côtés opposés de ladite ligne centrale A, ladite première patte (63', 63") ayant un premier alésage (65', 65"), et ladite seconde patte (64', 64") ayant un second alésage (66', 66") muni d'un filetage intérieur ; et
- chacune desdites pièces de verrouillage mâles (71, 72) est agencée sur la ligne centrale A et est conçue pour être reçue dans ledit espace (67', 67") entre lesdites première et seconde pattes (63', 63", 64', 64") desdites pièces de verrouillage femelles (61, 62) ; et
- chacun desdits éléments de verrouillage (81', 81") a une première extrémité munie d'un filetage extérieur (82', 82"), une seconde extrémité munie d'une tête en forme de cône (87', 87"), et une section médiane (85', 85") disposée entre lesdites première et seconde extrémités, lesdits éléments de verrouillage (81', 81") sont conçus pour être reçus dans lesdits premier et second alésages (65', 65", 66', 66") de ladite pièce de verrouillage femelle (61, 62).

7. Dispositif de raccord vasculaire selon la revendication 6, dans lequel les deux desdits éléments de verrouillage (81', 81") sont conçus pour être entrés dans lesdits premiers alésages (65', 65") à partir d'un premier côté et perpendiculairement à ladite ligne centrale A, pour croiser ladite ligne centrale A et ensuite être reçu dans lesdits seconds alésages (66', 66") munis de filetages intérieurs et agencés sur un second côté de ladite ligne centrale A opposé audit premier côté.

8. Dispositif de raccord vasculaire selon l'une quelconque des revendications précédentes, dans lequel chacune desdites pièces de verrouillage mâles (71, 72) comprend un œillet (73, 74) pourvu d'une fente ouverte (75', 75") faisant face audit côté de raccord dudit second élément de raccord (22).

9. Dispositif de raccord vasculaire selon la revendication 8, dans lequel lesdites sections médianes (85', 85") desdits éléments de verrouillage (81', 81") sont conçues pour être reçues dans lesdites fentes ouvertes (75', 75") prévues sur lesdits œillets (73, 74) desdites pièces de verrouillage mâles (71, 72) lorsque lesdites pièces de verrouillage mâles (71, 72) sont agencées dans lesdits espaces (67', 67") entre lesdites première et seconde pattes (63', 63", 64', 64") desdites pièces de verrouillage femelles (61, 62).

10. Dispositif de raccord vasculaire selon les revendications 8 et 9, dans lequel des premier et second évidements (76, 77) sont agencés autour des périmètres intérieurs desdits œillets (73, 74) et du même côté de ladite ligne centrale A desdites parties de verrouillage mâles (71, 72), lesdits premier et second évidements (76, 77) étant conçus pour recevoir lesdites têtes en forme de cône (87', 87") desdits éléments de verrouillage (81', 81").

11. Dispositif de raccord vasculaire selon la revendication 10, dans lequel ledit dispositif vasculaire (10) est dans une configuration verrouillée lorsque lesdits filetages extérieurs (82', 82") desdits éléments de verrouillage (81', 81") sont reçus et joints avec lesdits filetages intérieurs prévus dans lesdits seconds alésages (66', 66") desdites parties de verrouillage femelles (61, 62), et lesdites têtes en forme de cône (87', 87") desdits éléments de verrouillage (81', 81") sont reçus dans lesdits évidements (76, 77) prévus sur lesdits œillets (73, 74) agencés sur lesdites pièces de verrouillage mâles (71, 72).

12. Dispositif de raccord vasculaire selon l'une quelconque des revendications précédentes, dans lequel lesdits premier et second éléments saillants (93, 94) sont agencés sur un périmètre extérieur dudit second élément de raccord (22),

13. Dispositif de raccord vasculaire selon l'une quelconque des revendications 10 à 11 ou la revendication 12 lorsqu'elle dépend de la revendication 10 ou 11, dans lequel lesdits premier et second éléments saillants (93, 94) et lesdits évidements (76, 77) sont agencés sur des côtés opposés de ladite ligne centrale A dudit second élément de raccord (22).

14. Dispositif de raccord vasculaire selon l'une quelconque des revendications précédentes, dans lequel lesdites première et seconde parties découpées (91, 92) sont agencées sur un périmètre extérieur dudit premier élément de raccord (21).

15. Dispositif de raccord vasculaire selon l'une quelconque des revendications précédentes, dans lequel un sac d'enveloppement (98) est fixé à la surface externe (23', 23") du dispositif de raccord et reçoit et enferme la pompe cardiaque artificielle (50) pour protéger la pompe de toute croissance tissulaire.

16. Dispositif de raccord vasculaire selon l'une quelconque des revendications précédentes, dans lequel des premier et second moyens de réception (95, 96) sont disposés sur les premier et second éléments de raccord (21, 22) respectivement, les premier et second moyens de réception (95, 96) étant tout deux disposés du même côté de la ligne centrale A et sont adaptés pour faciliter la manipulation dudit dispositif de raccord vasculaire (10) lors de l'implantation.

17. Cœur artificiel total (TAH) (50) comprenant un premier et un second canal d'entrée (51, 52) et un premier et un second canal de sortie (53, 54), dans lequel un ou plusieurs des premier et second canaux d'entrée et de sortie (51, 52, 53, 54) sont raccordés à un dispositif de raccord vasculaire (10) selon l'une quelconque des revendications 1 à 16.
